# EUROPÄISCHE PATENTSCHRIFT

(11) **EP 2 658 603 B1**
(45) Veröffentlichungstag und Bekanntmachung des Hinweises auf die Patenterteilung: **15.06.2016**
(21) Anmeldenummer: 11799443.4
(22) Anmeldetag: 21.12.2011
(51) Int. Cl.: A61N 1/36, A61N 1/32

(54) **VORRICHTUNG ZUR NICHT-INVASIVEN, ELEKTRISCHEN TIEFENHIRNSTIMULATION**
DEVICE FOR NON-INVASIVE, ELECTRICAL DEEP-BRAIN STIMULATION
DISPOSITIF POUR LA STIMULATION ÉLECTRIQUE PROFONDE ET NON INVASIVE DU CERVEAU

(30) Priorität: 28.12.2010 DE 102010056433; 05.12.2011 DE 102011120213
(43) Veröffentlichungstag der Anmeldung: 06.11.2013
(73) Patentinhaber: EBS Technologies GmbH, 14532 Kleinmachnow (DE)
(72) Erfinder: PAULUS, Walter, 37075 Göttingen (DE); WARSCHEWSKE, Udo, 12249 Berlin (DE)
(74) Vertreter: Kruspig, Volkmar
(86) Internationale Anmeldenummer: PCT/EP2011/073619
(87) Internationale Veröffentlichungsnummer: WO 2012/089588

(56) Entgegenhaltungen:
- US-A1- 2006 173 510
- US-A1- 2009 287 108
- ANTAL A ET AL: "Comparatively weak after-effects of transcranial alternating current stimulation (tACS) on cortical excitability in humans", BRAIN STIMULATION,, Bd. 1, Nr. 2, 1. April 2008 (2008-04-01), Seiten 97-105, XP022674747, ISSN: 1935-861X, DOI: 10.1016/J.BRS.2007.10.001 [gefunden am 2007-12-03]
- PAULUS ET AL: "On the difficulties of separating retinal from cortical origins of phosphenes when using transcranial alternating current stimulation (tACS)", CLINICAL NEUROPHYSIOLOGY, ELSEVIER SCIENCE, IE, Bd. 121, Nr. 7, 1. Juli 2010 (2010-07-01), Seiten 987-991, XP027064559, ISSN: 1388-2457, DOI: 10.1016/J.CLINPH.2010.01.029 [gefunden am 2010-02-23]
- ZAGHI S ET AL: "Inhibition of motor cortex excitability with 15Hz transcranial alternating current stimulation (tACS)", NEUROSCIENCE LETTERS, LIMERICK, IE, Bd. 479, Nr. 3, 2. August 2010 (2010-08-02), Seiten 211-214, XP027112486, ISSN: 0304-3940 [gefunden am 2010-05-27]
- KANAI R ET AL: "Transcranial alternating current stimulation (tACS) modulates cortical excitability as assessed by TMS-induced phosphene thresholds", CLINICAL NEUROPHYSIOLOGY, ELSEVIER SCIENCE, IE, Bd. 121, Nr. 9, 1. September 2010 (2010-09-01), Seiten 1551-1554, XP027188278, ISSN: 1388-2457 [gefunden am 2010-04-09]
- SCHUTTER D J L G ET AL: "Retinal origin of phosphenes to transcranial alternating current stimulation", CLINICAL NEUROPHYSIOLOGY, ELSEVIER SCIENCE, IE, Bd. 121, Nr. 7, 1. Juli 2010 (2010-07-01), Seiten 1080-1084, XP027064573, ISSN: 1388-2457, DOI: 10.1016/J.CLINPH.2009.10.038 [gefunden am 2010-02-25]

## Beschreibung

Die vorliegende Erfindung betrifft eine Vorrichtung und ein Verfahren zur transkraniellen, nicht-invasiven, elektrischen Tiefenhirnstimulation wie sie insbesondere bei der Behandlung von neurologischen und psychiatrischen Erkrankungen und von Störungen der motorischen/kognitiven Funktionen im menschlichen Gehirn zum Einsatz kommen.

Bei der elektrischen Tiefenhirnstimulation (engl. "Deep Brain Stimulation" (DBS)) handelt es sich allgemein um ein neurochirurgisches Präzisionsverfahren, bei dem durch Zuführen eines Stromes niedriger Amplitude Hirnstrukturen insbesondere krankhaft überaktive Kernregionen im Gehirn gehemmt und somit die behindernden Symptome wirksam gelindert werden können. Die genaue Funktions- und Wirkungsweise der Tiefenhirnstimulation ist bisher noch ungeklärt und daher Gegenstand intensiver Forschung.

Etablierte Anwendungsgebiete sind unter anderem die Parkinson-Krankheit, der Essentielle Tremor oder die Dystonie. In der klinischen Erprobung befinden sich Anwendungen in den Bereichen Epilepsie, Depression, Zwangsstörung, Cluster-Kopfschmerz und Tourette-Syndrom.

Nach dem Stand der Technik sind derzeit im Wesentlichen zwei grundlegende Verfahren für die elektrische Tiefenhirnstimulation zu unterscheiden, nämlich die invasive Tiefenhirnstimulation und die nicht-invasive Tiefenhirnstimulation.

Die invasive Tiefenhirnstimulation ist nach dem Stand der Technik ein neurochirurgischer Eingriff in das Gehirn, mit dem krankheitsbedingte Fehlleistungen korrigiert werden sollen. Für die chronische Hirnstimulation werden dem Patienten meist eine oder zwei dünne Elektroden implantiert, die über subkutan verlegte Kabel mit einem Impulsgeber im Bereich der Brust oder dem Oberbauch verbunden sind. Dieser Impulsgeber gibt dauerhaft elektrische Impulse an die Zielregion im Gehirn ab, wodurch diese - je nach Stromfrequenz - entweder deaktiviert oder stimuliert werden kann. Da die elektrischen Impulse bei der invasiven Tiefenhirnstimulation dauerhaft abgegeben werden, wird dieses Verfahren auch als chronische Tiefenhirnstimulation bezeichnet; die dazugehörigen medizinischen Vorrichtungen sind unter der verbreiteten Bezeichnung "Hirnschrittmacher" bekannt.

Bislang wurden weltweit ca. 75.000 Patienten mit einem Hirnschrittmacher operativ versorgt. Prospektive kontrollierte und randomisierte Studien der letzten Jahre belegen die anhaltende Wirksamkeit des Therapieverfahrens im individuellen Krankheitsverlauf. Dabei werden nicht nur die Krankheitssymptome wie Zittern (Tremor), Steifigkeit (Rigor) und Bewegungsarmut (Bradykinese) gebessert, sondern nachweislich auch in ganzheitlicher Hinsicht die Lebensqualität.

Bei der operativen Behandlung durch Tiefenhirnstimulation, die auch als "stereotaktische" Operation bezeichnet wird, werden die Elektroden üblicherweise über ein kleines Bohrloch im Schädel der zu behandelten Person in das Gehirn eingebracht und zielgenau im Bereich des zu stimulierenden Hirnareals platziert. Somit ist es möglich, an vorher präzise festgelegten Stellen im Gehirn die Hirnaktivität durch elektrische Stimulation gezielt und dauerhaft bzw. chronisch zu manipulieren.

Neben den hohen Kosten solcher invasiven Eingriffe am menschlichen Gehirn sind es jedoch vor allen Dingen gesundheitliche Risiken wie etwa das Auftreten von Hirnblutungen oder Gehirninfektionen, die es abzuwägen gilt. Dabei ist zu bedenken, dass gesundheitliche Risiken nicht nur unmittelbar durch den chirurgischen Eingriff selbst sondern aufgrund der Implantate auch noch während der gesamten Behandlungsdauer latent vorhanden sind. Vorteil der invasiven Stimulationsverfahren sind die Präzision, mit der die entsprechenden Gehirnareale gezielt einer Tiefenhirnstimulationsbehandlung zugeführt werden können sowie die Möglichkeit einer chronischen bzw. dauerhaften Behandlung.

Die nicht-invasiven Verfahren auf dem Gebiet der elektrischen Neurostimulation von Hirnarealen betreffen die sog. transkraniellen Verfahren zur nicht-invasiven Tiefenhirnbehandlung, insbesondere die transkranielle Gleichstromstimulation (tDCS) und die transkranielle Wechselstromstimulation (tACS), bei denen eine therapeutische Stimulation ohne einen interventionellen Eingriff in das Gehirn auskommt. Stattdessen erfolgt die Behandlung "transkraniell", d.h. von außerhalb des Schädels "durch den Schädel hindurch". Sowohl bei der transkraniellen Gleichstromstimulation (tDCS) als auch bei der transkraniellen Wechselstromstimulation (tACS) werden zwei Elektroden auf der Kopfhaut des Patienten platziert und für eine begrenzte Zeitdauer von bis zu 15 Minuten zur Stimulation der neuronalen Schaltkreise im Gehirn mit einem Gleich- bzw. Wechselstrom beaufschlagt.

Aus der US4856526 ist ein Verfahren zur transkraniellen Wechselstromstimulation zur Minderung von Kopfschmerzen bekannt, bei dem über zwei am Kopf des Patienten platzierte Elektroden eine hochfrequente Wechselspannung appliziert wird. Der Frequenzbereich der Wechselspannung liegt im Bereich von 12 bis 20 kHz. Für die Behandlung werden eine erste Elektrode auf einer Kopfseite und eine weitere Elektrode auf der gegenüberliegenden Kopfseite platziert.

Die DE 102008043973 A1 offenbart eine Vorrichtung zur transkraniellen Gleichstromstimulation (tDCS), die bei der Behandlung bzw. Stimulation von neuronalen Hirnstrukturen zum Einsatz kommt. Bei diesem Verfahren wird über eine großflächige Elektrodenanordnung, die z.B. matrixartig in einer Kappe befindlich ist, auf der Kopfhaut ein schwacher kontinuierlicher Gleichstrom appliziert. Zur Einstellung einer optimalen Elektrodenfläche, mit der eine Fokussierung der induzierten Gesamtladung im Zentralnervensystem möglich ist, können einzelne Elektrodenpaare dieser rasterartig angeordneten Menge von Flächenelektroden zu Stimulationszwecken angeregt werden. Die Ermittlung dieser optimalen Elektrodenfläche erfolgt unter Berücksichtigung des Übergangswiderstands zwischen der Elektrodenfläche und der Kopfhaut. Durch selektive Ansteuerung von einzelnen Elektrodenbereichen der rasterartigen Matrix ist die gesamte zusammenhängende Elektrodenfläche zur Applizierung eines Gleichstromes variabel einstellbar. D.h. die genutzte Elektrodenfläche kann größer oder kleiner gewählt werden, um ein zwischen der Elektrodenanordnung befindliches Gehirnareal mehr oder weniger zu stimulieren (Fokussierung der induzierten Gesamtladung).

US-A1-2006/173510 offenbart eine Vorrichtung zur Stimulation von tiefen Hirnarealen, die wenigstens einen Signalgenerator zur Erzeugung eines elektrischen Wechselstromsignals, eine Elektroden-Anordnung, die an dem Kopf einer zu behandelnden Person platzierbar und mit dem Signalgenerator elektrisch verbindbar ist, um ein Wechselstromsignal zu applizieren, aufweist. Mittels der Elektroden-Anordnung sind wenigstens zwei Wechselstromsignale applizierbar. Das Wechselstromsignal hat eine Frequenz von zwischen 0.1 Hz und 2.5 KHz.

"Comparatively weak after-effects of transcranial alternating current stimulation (tACS) on cortical excitability in humans" (Brain Stimulation, Mai 2008, Stiten 97-105) offenbart eine Vorrichtung zur Stimulation von tiefen Hirnarealen mittels einer transkraniellen Wechselstromstimulation (tACS).

Da im Gegensatz zur invasiven Tiefenhirnstimulation bei der nicht-invasiven, transkraniellen elektrischen Tiefenhirnstimulation unter Berücksichtigung eines höheren Widerstands, d.h. Übergangswiderstand zwischen Elektroden und Kopf bzw. Kopfhaut des Patienten, ein höherer Stimulationsstrom appliziert werden muss, um in dem Hirnareal die für eine erfolgreiche Behandlung erforderliche Gesamtladung zuzuführen, ist bei der Gleichstromstimulation aus Sicherheitsgründen die Behandlungsdauer auf in etwa 15 Minuten begrenzt.

Bei der transkraniellen Gleichstromstimulation (tDCS) konnten im Hinblick auf die Stimulationsbehandlung Langzeiteffekte festgestellt werden. Nach dem Abschalten des aktiven Stimulationsstromes bewirkt eine Gleichstromstimulation von etwa 10 Minuten eine Erregbarkeitserhöhung in dem behandelten Gehirnareal von bis zu einer Stunde, und eine Gleichstromstimulation von etwa 15 Minuten bewirkt in dem behandelten Gehirnareal eine Erregbarkeitserhöhung von bis zu zwei Stunden.

Die Studie von Park et. al. (NOVEL ARRAY-TYPE TRANSCRANIAL DIRECT CURRENT STIMULATION (tDCS) SYSTEM) befasst sich mit Verfahren zur Erhöhung der Dichte/Intensität des Stimulationsgleichstroms in dem zu behandelnden Gehirnareal. Bei einer ersten Lösung werden zwei Elektroden so an dem Kopf platziert, dass die Trajektorie des Stimulationsgleichstroms genau durch das zu behandelnde Gehirnareal verläuft. Bei einer zweiten Lösung werden zwei Elektrodenarrays verwendet, die in einer räumlichen Anordnung mehrere Elektrodenpaare bilden, wobei einander gegenüberliegende Elektroden jeweils ein Elektrodenpaar bilden. Die einzelnen Elektrodenpaare können wiederum mit unterschiedlichen Gleichstromsignalen angesteuert werden. Zur Erreichung der optimalen Stimulationsstromdichte in dem zu behandelnden Gehirnareal werden anhand eines Schädelmodells die Widerstandsverteilungen ermittelt und auf deren Grundlage die für eine verbesserte Stimulationsstromdichte an den einzelnen Elektrodenpaaren zu injizierenden Stromstärken ermittelt. Die Ermittlung der optimalen injizierten Stromverteilungen basiert auf dem Überlagerungsprinzip.

Ein entscheidender Nachteil der Gleichstromstimulation (tDCS) besteht darin, dass durch die Verwendung von Flächenelektroden der maximale Wirkbereich des Gleichstromes nicht an den Orten der Elektroden erfolgt, so dass eine kortikal lokal fokussierte Therapie nur bedingt möglich ist.

Ein weiterer Nachteil besteht darin, dass bei Applizierung eines Stromes Trajektorien entstehen, die im Wesentlichen parallel oder zumindest nebeneinander liegend verlaufen. Zwar werden somit die Stromdichten in dem zu behandelnden Hirnareal erhöht, jedoch werden nach wie vor verstärkt Hirnareale mitbehandelt, die an dem zur Behandlung beabsichtigten Zielareal unmittelbar angrenzen. Eine gezielte Stimulationsbehandlung ist daher nur eingeschränkt möglich.

Schließlich ist die maximale durchgehende Behandlungsdauer bei den bekannten Verfahren und Vorrichtungen der nicht-invasiven elektrischen Tiefenhirnstimulation nach wie vor unzufriedenstellend. Es besteht daher ein Bedürfnis nach einem Verfahren sowie einer Vorrichtung zur transkraniellen elektrischen Tiefenhirnstimulation, bei denen längere Behandlungsphasen möglich sind.

Es ist daher die Aufgabe der vorliegenden Erfindung, eine Vorrichtung sowie ein Verfahren zur nicht-invasiven, elektrischen Tiefenhirnstimulation zu schaffen, mit welchen eine zielgenaue und effektive Behandlung von Hirnarealen einfach und kostengünstig realisierbar ist.

Diese Aufgabe wird durch die Merkmale der unabhängigen Ansprüche 1 und 13 gelöst. Vorteilhafte Ausgestaltungen ergeben sich aus den Unteransprüchen.

Erfindungsgemäß wird diese Aufgabe gelöst durch eine Vorrichtung zur transkraniellen Wechselstromstimulation (tASC) von tiefen Hirnarealen, aufweisend wenigstens einen Signalgenerator zur Erzeugung eines elektrischen Wechselstromsignals, eine Elektroden-Anordnung, die an dem Kopf einer zu behandelnden Person platzierbar und mit dem Signalgenerator elektrisch verbindbar ist, um ein Wechselstromsignal zu applizieren, wobei mittels der Elektroden-Anordnung wenigstens zwei Wechselstromsignale applizierbar sind, deren Trajektorien das zu behandelnde Hirnareal bzw. die Zielregion kreuzen. Als Trajektorien werden hierin Raumkurven verstanden, entlang welcher die Wechselströme das Gehirn einer zu behandelnden Person durchqueren.

Es wird hierbei die erfindungsgemäße Erkenntnis genutzt, dass nahezu geradlinige Trajektorien vorliegen, wenn Wechselströme mit einer Frequenz im kHz-Bereich genutzt werden. Im Gegensatz hierzu fließen Wechselströme mit einer Frequenz von unter 200 Hz entlang der Nervenfasern, was eine genaue zielführende Behandlung erschwert.

Der Kerngedanke der Erfindung besteht darin, dass eine therapeutische Stimulation ohne einen invasiven interventionellen Eingriff in das Gehirn erfolgt und hierzu das betreffende funktionale Areal des Gehirns gezielt mit elektrischen Wechselströmen angeregt wird, wobei benachbarte Areale nicht oder nur wenig bzw. unterschwellig belastet werden. Dies wird erreicht durch eine spezielle räumliche Anordnung der Elektroden, zwischen denen die Elektrostimulation mittels Wechselstrom derart appliziert wird, dass die Stimulationstrajektorien das in der Tiefe zu stimulierende Areal kreuzen, wodurch eine Verdichtung des Stimulationsstromes und damit eine Fokussierung der Energiezufuhr im zu behandelnden Areal bewirkt wird. Es wurde festgestellt, dass für die Wirksamkeit einer Tiefenhirnstimulation u.a. die relative Stimulationsdifferenz zwischen der in der Zielregion und den umgebenden Hirnarealen injizierte Stromdichte maßgeblich ist. Je größer die relative Differenz der Stromdichten von Zielareal und an dem Zielareal angrenzenden Arealen, desto größer der Stimulationseffekt.

Weiterhin ergibt sich durch die Applizierung eines Wechselstromes - im Gegensatz zur Gleichstromapplikation - eine verbesserte Nachwirkung der Elektrostimulation. Es konnte zwar sowohl bei der transkraniellen Wechselstromstimulation (tACS) als auch bei der transkraniellen Gleichstromstimulation (tDCS) aufgezeigt werden, dass bei entsprechender kurzzeitiger, bis zu zehnminütiger Behandlung eine Beibehaltung des Stimulationseffektes nach dem Abschalten des aktiven Stimulationsstromes, d.h. eine gewisse Langzeitwirkung gegeben ist. Insgesamt konnte jedoch festgestellt werden, dass die transkranielle Wechselstromstimulation (tACS) gegenüber der transkranielle Gleichstromstimulation (tDCS) höhere Langzeiteffekte, d.h. eine Beibehaltung der Stimulationswirkung, bewirkt, was auf die zeitlich an- und abschwellende Stimulationswirkung durch den Wechselstrom zurückzuführen ist.

Des Weiteren kann die Elektroden-Anordnung derart an dem Kopf platziert sein und/oder der applizierte Wechselstrom in Bezug auf die Amplitude und/oder die Frequenz so eingestellt werden, dass sich die Trajektorien wenigstens zweier applizierter Wechselstromsignale in der Zielregion kreuzen. Insbesondere können sich die Trajektoren ausgehend von zwei verschiedenen Ebenen in der Zielregion, insbesondere in einem definierten Punkt kreuzen, der vorzugsweise im räumlichen Zentrum der Zielregion liegt, so dass der Überlagerungseffekt in der Zielregion weiter verstärkt wird, während die umgebenden Hirnregionen noch weniger belastet werden. Dadurch kann die Stimulationsdifferenz zwischen Zielregion und benachbarten Hirnregionen vergrößert und dementsprechend die Stimulationswirkung verbessert werden.

Vorzugsweise ist ein appliziertes Stromsignal ein hochfrequentes Wechselstromsignal mit einer Frequenz von zwischen 1 kHz und 50 kHz. Überraschenderweise führen derartige hochfrequente Wechselstromsignale zu einer erhöhten Stimulationswirkung in der Zielregion, was auf die erhöhte Empfindlichkeit neuronaler Netzwerke durch Modulation des elektrischen Feldes in der Zielregion zurückzuführen ist.

Das applizierte Wechselstromsignal ist vorzugsweise ein gepulstes Wechselstromsignal. Es können jedoch auch andere Wechselstromsignalformen wie z.B. Sinussignalform, Dreiecksignalform, Sägezahnsignalform, aber auch ein Rauschsignal appliziert werden. Da Reize im menschlichen Erregungsleitungssystem typischerweise in Form von elektrischen Impulsen übertragen werden, kann durch ein gepulstes Wechselstromsignal in vorteilhafter Weise eine Annäherung an die dem menschlichen Körper inhärente Kurvenform und damit eine verbesserte Erregungs- bzw. Stimulationswirkung erreicht werden. Die elektrischen Stimulationsparameter wie Frequenz, Amplitude, Kurvenform und Pulsfolge des Wechselstromsignals sowie die Dauer der Stimulation, die zeitliche Abfolge der Therapiesitzungen und die Größe und Lage des Zielareals können je nach Therapieansatz variiert werden.

Nach einer vorteilhaften Ausgestaltung umfasst die Elektroden-Anordnung wenigstens zwei Elektrodenpaare, welches derart räumlich positionierbar ist, dass die Trajektorien wenigstens zweier applizierter Stromsignale die Zielregion kreuzen oder sich in der Zielregion kreuzen und dort konzentrieren und verstärken.

Eine vorteilhafte Weiterbildung der Erfindung besteht darin, dass eine Stromapplikations-Steuerungseinrichtung zur Steuerung der an dem wenigstens einen Elektrodenpaar applizierten Stromsignale bereitgestellt ist, die derart betreibbar ist, dass die wenigstens zwei Stromsignale sequentiell, insbesondere wechselseitig, appliziert werden. Demzufolge werden nacheinander Stromsignale appliziert, die entlang verschiedener Trajektorien die Zielregion kreuzen, wodurch sich zwar in der Zielregion eine dauerhafte Stimulationswirkung ergibt, jedoch in den zur Zielregion benachbarten Hirnarealen stimulationsfreie Phasen entstehen, so dass die Erregungswirkung in diesen Bereichen weiter abgesenkt werden kann.

Vorteilhafterweise kann durch die Applizierung eines hochfrequenten Wechselstroms und der sequentiellen Ansteuerung eine erhöhte Stimulation im Zielareal bei gleichzeitig verminderter Erregungswirkung im umliegenden Hirnareal erreicht werden. Z.B. können insgesamt zwei Elektrodenpaare in verschiedenen räumlichen Anordnungen, d.h. einer ersten und einer zweiten Positionen an dem Kopf der zu behandelnden Person vorgesehen sein, wobei wechselseitig dem Elektrodenpaar an der ersten und der zweiten Position ein Stimulationswechselstrom zugeführt wird. Vorzugsweise werden die Wechselstromimpulse jeweils nur für eine sehr kurze Dauer appliziert.

Gemäß einer bevorzugten Ausführungsform der Lösung umfasst die ElektrodenAnordnung ein Elektrodenarray-Paar, wobei jedes Elektrodenarray eine Vielzahl von rasterartig bzw. matrixartig angeordneten Elektroden umfasst, wobei vorzugsweise ein Elektrodenarray als Zielelektrode und ein Elektrodenarray als Referenzelektrode fungiert, und wobei die Anzahl der Zielelektroden der Anzahl der Referenzelektroden entspricht. Es kann jedoch auch ein Elektrodenpaar aus einem Elektroden-Array, das z.B. die Zielseite bildet, und einer einzelnen Elektrode, die entsprechend die Referenzseite bildet, vorgesehen sein.

Im Falle der Verwendung von, z.B. vorgefertigten, Elektrodenarrays, werden diese räumlich mit der Zielregion korreliert. Danach werden die Paarungen Elektroden ermittelt und verwendet, deren Trajektorien die Zielregion kreuzen. Hierzu ist nach einer vorteilhaften Weiterbildung der Erfindung die Stromapplikations-Steuerungseinrichtung in der Lage, genau diejenigen Elektrodenpaarungen aus der Vielzahl von Elektroden auszuwählen und anzusteuern, deren Stromsignal-Trajektorien die Zielregion kreuzen, insbesondere deren Stromsignal-Trajektorien sich in der Zielregion kreuzen. Die Stromapplikations-Steuerungseinrichtung kann diese Elektrodenpaare in Abhängigkeit von dem zeitlichen Verlaufsmuster der Therapie und einer entsprechenden Vorauswahl von Elektrodenpaarungen, sequentiell ansteuern und aktivieren.

Die Elektrodenpaare können z.B. direkt an mehreren unabhängigen Stimulationssignalgeneratoren angeschlossen sein und unabhängig angesteuert werden. Bei einer bevorzugten Ausgestaltung umfasst die Stromapplikations-Steuerungseinrichtung einen Matrix-Schalter und eine Controller-Einrichtung, die wiederum den Matrixschalter ansteuert. Über den Matrixschalter kann ein frei wählbares Elektrodenpaar einem Signalgenerator oder Funktionsgenerator zugeordnet werden, wobei das Schalten der Elektroden mittels des Matrixschalters durch den Controller mit den Signalen eines oder mehrerer Signaloder Funktionsgeneratoren abgestimmt wird.

Die Fixierung eines oder mehrerer Elektrodenpaare oder der Elektrodenarrays in einer räumlichen Anordnung am Kopf der zu behandelnden Person erfolgt mit Hilfe eines Befestigungsmittels. Als Befestigungsmittel dient ein Gurt oder eine Haube, wobei die Elektrodenarrays oder die einzelnen Elektroden in dem jeweiligen Befestigungsmittel integriert sind. Im einfachsten Fall sind die Elektroden als Flächenelektroden, insbesondere als Pflaster-Elektroden, vorgesehen, die im Bereich der Kontaktfläche der Elektroden einen Haftabschnitt aufweisen.

Vorzugsweise sind die Elektroden der Elektrodenpaare flächig ausgebildet und haben vorzugsweise eine Fläche von einigen mm² bis cm².

Die Haube kann aus einem textilen Abstandsgewirke mit integrierten Elektroden bestehen.

Gemäß einer weiteren bevorzugten Ausführungsform besteht eine Möglichkeit des volumetrischen Stimulationskonzeptes darin, ein Elektrodenpaar z.B. mittels einer navigierbaren mechatronischen Einrichtung schnell und gezielt derart zu versetzen, dass es Positionen in einer bestimmten Abfolge einnimmt. So wird die Energiezufuhr auf das zu behandelnde Areal fokussiert und umgebende Bereiche des Gehirns nur geringfügig beeinträchtigt. Hierzu umfasst die Stromapplikations-Steuerungseinrichtung eine Positioniervorrichtung, an welcher ein Elektrodenpaar vorgesehen ist, wobei die Positioniervorrichtung das Elektrodenpaar an dem Kopf der zu behandelnden Person in einer ersten Elektrodenpaar-Stellung sowie in einer zweiten Elektrodenpaar-Stellung hält, wobei die erste und die zweite räumliche Elektrodenpaar-Stellung derart zueinander versetzt sind, dass die Trajektorien der in der ersten und in der zweiten Elektrodenpaar-Stellung applizierten Stromsignale die Zielregion kreuzen oder sich die Trajektorien dieser Stromsignale in der Zielregion kreuzen. Die Stromapplikations-Steuerungseinrichtung umfasst zudem eine Controller-Einrichtung, die den Betrieb der Positioniervorrichtung sowie den Betrieb des Funktionsgenerators vorgibt, um die Positionierung des Elektrodenpaares und die Stromapplizierung in den verschiedenen Elektrodenpaar-Stellungen abzustimmen.

Eine vorteilhafte Weiterbildung der Erfindung besteht darin, dass die Lage und Ausmaße der Zielregion auf einer Planungsstation bestimmt werden. Grundlage für die Zielplanung sind z.B. volumetrische anatomische Bilddaten wie z.B. CT oder MRT. Zusätzlich können die Areale auch mittels funktioneller Hirnatlanten (z.B. Atlas von Schaltenbrand-Wahren) abgegrenzt werden. Weitere Planungsmöglichkeiten basieren auf der Nutzung räumlicher funktioneller Daten, wie z.B. fMRT, 3D EEG² oder der Impedanztomographie. Es ist auch möglich, mehrere Datensätze miteinander zu korrelieren (z.B. mit der multimodalen Bilddatenfusion).

Die räumliche Korrelation der Elektrodenpaare mit dem Patientenhirn bzw. der Zielregion kann z.B. mittels bildgestützter Navigation erfolgen. Dabei werden die räumlichen Daten bzgl. der Zielregion mit dem Positionssystem des Navigationssystems abgeglichen und um die Positionen der Elektrodenpaare erweitert. Bei Verwendung von vorkonfigurierten Elektrodenarrays können diese z.B. mittels Abformmasse oder geeigneter Bissplatten am Kopf befestigt werden, so dass bei erneuter Verwendung die Passform eine akkurate Korrelation mit der Zielregion sicherstellt und keine erneute Korrelation der Elektroden mit der Zielregion erforderlich ist.

Die räumliche Korrelation von Elektrodenposition und Zielgebiet kann auch ohne das Verwenden eines zusätzlichen Positionsmesssystems erfolgen. Dazu werden die Elektroden direkt in einem Datensatz lokalisiert (z.B. durch Bilddatenanalyse bei Verwendung geeigneter CT oder MRT Marker an den Elektroden oder Elektrodensockeln, durch das Referenzieren auf Elektroden der 3D EEG Ableitung oder Impedanztomographie). In einem zweiten Schritt werden Stimulationssequenzen unter Verwendung geeigneter Elektrodenpaarungen festgelegt und für das Therapieziel geeignete Stimulationsparameter gewählt.

Zusätzlich ist eine Feedback-Einrichtung vorgesehen, um das Biofeedback des Patienten aufzunehmen und den Therapieerfolg bewerten zu können oder ggf. Gefährdungen des Patienten (z.B. epileptischer Anfall) zu erkennen. Die Feedbackdaten können in einem von dem Controller überwachten Regelkreis zur Optimierung der Stimulationsparameter verwendet werden.

Die beschriebene und in den Ansprüchen 1 bis 13 beanspruchte Vorrichtung findet Anwendung in Verfahren zur Behandlung von Funktionsstörungen des Gehirns, insbesondere zur therapeutischen Behandlung der Parkinson-Krankheit, insbesondere von Zittern (Tremor), Steifigkeit (Rigor), Bewegungsarmut (Bradykinese), Schlaganfall, Lähmungen, Depression, Schizophrenie, Zwangsstörungen, Angsterkrankungen und Panikstörungen, Demenz, fokalen neuropsychologischen Defiziten, Multipler Sklerose, Restless legs Syndrom, Schmerzen, Kopfschmerz, Migräne, der Dystonie, der Epilepsie, und des Tourette-Syndroms.

Es wird weiterhin ein Verfahren vorgeschlagen zur transkraniellen, nicht invasiven, Wechselstromstimulation (tACS) von tiefen Hirnarealen umfassend folgende Schritte:

Platzieren wenigstens zweier Elektrodenpaare an Positionen am Kopf einer zu behandelnden Person, an denen die Trajektorien der applizierten Wechselstromsignale das zu behandelnde Hirnareal bzw. die Zielregion kreuzen oder die Trajektorien der Wechselstromsignale sich in der Zielregion kreuzen; oder Platzieren eines Elektrodenarray-Paares in Korrelation mit der zu behandelnden Zielregion am Kopf einer zu behandelnden Person und Ermitteln der Paarungen von Elektroden, deren Trajektorien die Zielregion kreuzen oder deren Trajektorien sich in der Zielregion kreuzen; Anschließen der Elektrodenpaare an die Wechselstromquelle; und Applizieren eines Wechselstromsignals an den Elektroden zur Stimulierung der Zielregion.

Vorzugsweise werden die Elektrodenpaare gleichzeitig oder sequentiell bzw. nacheinander, insbesondere wechselseitig mit einem Wechselstromsignal beaufschlagt. Vorzugsweise wird das Wechselstromsignal auf eine Frequenz von zwischen 1 kHz und 50 kHz eingestellt und/oder das Wechselstromsignal als gepulstes Wechselstromsignal appliziert und/oder die elektrischen Stimulationsparameter wie Frequenz, Amplitude, Kurvenform und Pulsfolge variiert.

Vorzugsweise werden die zu stimulierenden Hirnareale vor der Stimulationsbehandlung bestimmt, insbesondere deren Lage und Ausmaße, insbesondere mittels volumetrischer anatomischer Bilddaten wie z.B. CT oder MRT.

Es wird zudem ein Verfahren vorgeschlagen zur transkraniellen, nicht invasiven, Wechselstromstimulation (tACS) von tiefen Hirnarealen umfassend die Schritte: Bereitstellen eines Elektrodenpaares und Anschließen des Elektrodenpaares an die Wechselstromquelle; Nacheinander Platzieren des Elektrodenpaares wenigstens an zwei verschiedenen Positionen an dem Kopf einer zu behandelnden Person, wobei die Positionen derart ausgewählt werden, dass die zu erwartenden Trajektorien der applizierten Wechselstromsignale die Zielregion kreuzen oder sich in der Zielregion kreuzen, und Applizieren eines Wechselstromsignals an den mehreren Position zur Stimulierung des tiefen Hirnareals.

Vorzugsweise wird der Schritt des nacheinander Platzierens der Gesamtbehandlungszeit entsprechend lange wiederholt. Vorzugsweise wird das Wechselstromsignal auf eine Frequenz von zwischen 1 kHz und 50 kHz eingestellt und/oder das Wechselstromsignal als gepulstes Wechselstromsignal appliziert und/oder die elektrischen Stimulationsparameter wie Frequenz, Amplitude, Kurvenform und Pulsfolge variiert. Vorzugsweise werden die zu stimulierenden Hirnareale vor der Stimulationsbehandlung bestimmt, insbesondere deren Lage und Ausmaße, wiederum insbesondere mittels volumetrischer anatomischer Bilddaten wie z.B. CT oder MRT.

Weitere Merkmale und Vorteile der Erfindung ergeben sich aus den Zeichnungen, in denen
- Fig. 1: ein schematisches Blockschaltbild eines Tiefenhirnstimulators gemäß einer ersten bevorzugten Ausführungsform zeigt;
- Fig. 2: eine Ansicht (von oben) der Elektroden-Anordnung zeigt, die gemäß einer ersten Variante der lokal fokussierten Therapie betrieben wird;
- Fig. 3: eine Ansicht (von oben) der Elektroden-Anordnung zeigt, die gemäß einer zweiten Variante der lokal fokussierten Therapie betrieben wird;
- Fig. 4: eine Ansicht (von oben) der Elektroden-Anordnung zeigt, die gemäß einer dritten Variante der lokal fokussierten Therapie betrieben wird;
- Fig. 5: ein Elektrodenarray-Paar gemäß der in Fig. 4 gezeigten Therapievariante schematisch in einer räumlichen Anordnung zeigt; und
- Fig. 6: eine Prinzipdarstellung eines Tiefenhirnstimulators mit Positioniervorrichtung gemäß einer zweiten bevorzugten Ausführungsform zeigt.

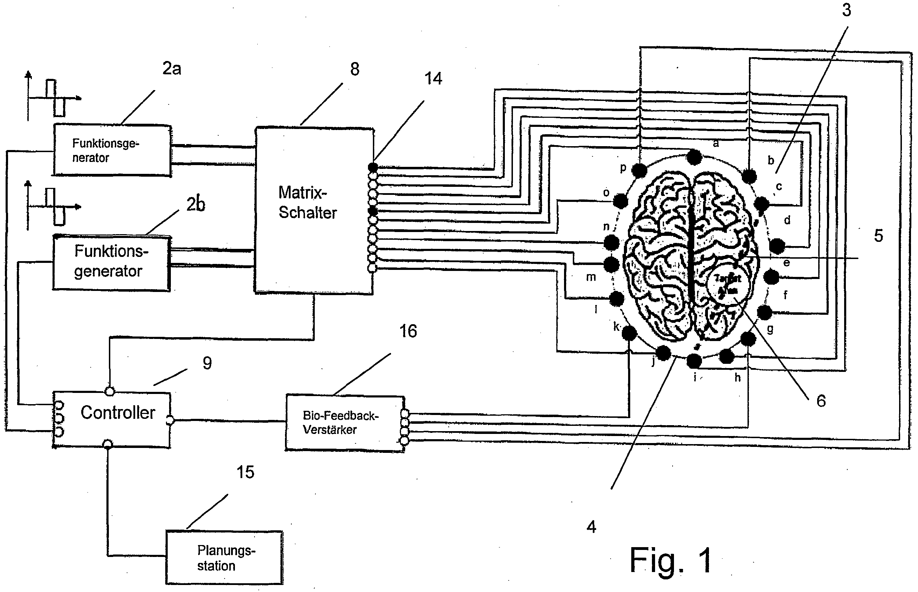

Fig. 1 zeigt eine erste bevorzugte Ausführungsform einer Vorrichtung zur transkraniellen Wechselstromstimulation (tASC) von tiefen Hirnarealen, die im folgenden als Tiefenhirnstimulator bezeichnet wird und mit dem Bezugszeichen 1 angegeben ist. Der Tiefenhirnstimulator 1 umfasst einen oder mehrere unabhängige Signalgeneratoren 2a, 2b zur Erzeugung, z.B. gepulster, elektrischer Wechselströme.

Als Signalgeneratoren werden ein oder mehrere handelsübliche Funktionsgeneratoren verwendet, bei denen die Stimulationssignale bezüglich Frequenz, Amplitude, Kurvenform und Pulsfolgen frei modulierbar sind.

Weiterhin umfasst der Tiefenhirnstimulator 1 eine Elektroden-Anordnung 3 mit einer Vielzahl von Elektroden 3a-3p, die über die Außenumfangsfläche des Kopfes 4 einer zu behandelnden Person verteilt angeordnet sind. Wie in der Ansicht von oben des Kopfes 4 zu sehen ist, sind Elektroden an sämtlichen Bereichen des Kopfes 4, d.h. Vorderseite, Rückseite und seitlichen Bereichen befindlich.

Ein Matrix-Schalter 8 ist zwischen der Elektroden-Anordnung 3 und den Funktionsgeneratoren 2a, 2b angeordnet und enthält eine Matrixschaltung mit insgesamt zwölf Ausgangsanschlüssen 14, die jeweils einer Elektrode 3a-3p zugeordnet und mit dieser elektrisch verbunden sind. Jeder Ausgangsanschluss 14 kann als ein Minus- oder ein Pluspol fungieren. Der Matrix-Schalter 8 ordnet jedem ausgewählten Elektrodenpaar Signale aus einem Funktionsgenerator zu, stellt eine elektrische Verbindung her und unterbricht diese wieder. In Fig. 1 wählt der Matrix-Schalter 8 genau solche Paarungen von Elektroden 3a-3p aus, deren Trajektorie 5 das zu behandelnde Hirnareal bzw. die Zielregion 6 kreuzt, d.h. in Fig. 1 z.B. die Paarung 3c-3i, und stellt eine Verbindung zu dem Funktionsgenerator 2a her, so dass die ausgewählte Elektrodenpaarung 3c-3i mit einer gepulsten Wechselspannung beaufschlagt wird, um einen Stromfluss durch die Zielregion 6 und eine entsprechende Stimulierung dieser Region zu bewirken.

Die Auswahl der Elektrodenpaarungen, die Zuordnung zwischen den Elektrodenpaarungen und den Funktionsgeneratoren 2a, 2b,... sowie die Steuerung des Matrix-Schalters 8 erfolgt durch einen Controller 9. Unter Controller wird eine Steuereinheit verstanden, die verschiedene Abläufe steuert oder regelt. Bei dem vorliegenden Controller handelt es sich um einen Mikrocontroller.

Der Controller 9 ist darüber hinaus mit den Funktionsgeneratoren 2a, 2b elektrisch verbunden. Das Schalten der Elektrodenpaare 3a-3p mittels des Matrixschalters 8 kann durch den Controller 9 mit den Signalen eines oder mehrerer Funktionsgeneratoren 2a, 2b abgestimmt werden. Je nach Therapieansatz können beispielsweise wechselseitig verschiedene Wechselstromsignale, die von verschiedenen Funktionsgeneratoren bereitgestellt werden, appliziert werden oder gleichzeitig über mehrere ausgewählte Elektrodenpaarungen jeweils nur ein Wechselstromsignal eines Funktionsgenerators appliziert werden.

Wie in Fig. 1 zu sehen, ist am Controller 9 eine Planungsstation 15 angeschlossen, welche die Lage und die Ausmaße der Zielregion 6 sowie die Stimulationsparameter wie etwa Signalform und Signalfrequenz bestimmt. Die Planungsstation 15 besitzt eine Funktion zur Auswahl der Elektroden entsprechend der Zielregion 6. Grundlage für die Zielplanung sind z.B. volumetrische anatomische Bilddaten wie z.B. CT oder MRT. Zusätzlich können die Areale auch mittels funktioneller Hirnatlanten (z.B. Atlas von Schaltenbrand-Wahren) abgegrenzt werden. Weitere Planungsmöglichkeiten basieren auf der Nutzung räumlicher funktioneller Daten, wie z.B. fMRT, 3D EEG² oder der Impedanztomographie, wie nachfolgend näher beschrieben. Es ist auch möglich mehrere Datensätze miteinander zu korrelieren (z.B. mit der multimodalen Bilddatenfusion).

Die Elektrische Impedanz-Tomografie (EIT) ist ein nicht-invasives bildgebendes Verfahren, bei dem auf Grundlage der Leitfähigkeitsverteilung im Gehirn eine tomografische Abbildung desselben erhalten wird. Vorteilhafterweise können die an dem Kopf 4 platzierten Elektrodenpaare 3a-3p als Sensorelemente verwendet werden, wobei an einem Elektrodenpaar, z.B. 3a/3i, ein Messstrom zugeführt und eine elektrische Potentialverteilung an den anderen Elektrodenpaaren 3b-3h & 3j-3p bestimmbar ist. Die EIT nutzt die Leitfähigkeitsverteilung in einem Körper, die u.a. auch von physiologischen Parametern abhängig ist. Mit der Impedanztomografie können somit Erkenntnisse betreffend die Morphologie und die Funktion gewonnen werden. Die verwendeten Messströme sind höherfrequente Wechselströme im Frequenzbereich von 10 bis 100 kHz und haben eine geringe Stromstärke im Bereich von 1 bis 10 Milliampere, so dass in der Messphase keine Stimulation stattfindet.

Gemäß einem erfindungsgemäßen Ansatz besteht die Möglichkeit, das Verfahren der Impedanz-Tomografie als Feedbackanalyse der erfindungsgemäßen Stimulation zu nutzen. Das heißt, es wird die Wechselstromstimulation ausgelöst und gleichzeitig der Widerstand bestimmt. Die Stimulation erregt hierbei die Nervenzellen, wodurch der Widerstand geringer wird. Die Stimulationsintensität und/oder die Frequenz können dann entsprechend angepasst werden.

Zusätzlich verfügt der Tiefenhirnstimulator 1 über die Möglichkeit, mittels Biofeedback den Therapieerfolg zu bewerten oder ggf. Gefährdungen des Patienten (z.B. epileptischer Anfall) zu erkennen. Hierzu ist ein Feedbackdaten-Rückführungszweig vorgesehen, in welchem ein Bio-Feedback-Verstärker 16 zwischen den platzierten Sensor-Elektroden und dem Controller 9 geschaltet ist. Somit entsteht ein Regelkreis zur Optimierung der Stimulationsparameter, der von dem Controller 9, der das jeweilige Therapie-Programm vorgibt, überwacht wird.

Im Folgenden werden der Betrieb und die Arbeitsweise des Tiefenhirnstimulators 1 vor dem Hintergrund eines diagnostizierten Krankheitsbildes näher beschrieben.

Zunächst werden die Elektroden am Kopf der zu behandelnden Person platziert. Hierzu werden vorteilhafter Weise eine Vielzahl von Elektroden verwendet, die entweder in einem Gurt oder einer Haube (nicht in der Figur zu sehen) integriert sind. Der Elektrodengurt bzw. die Elektrodenhaube kann ohne Berücksichtigung der genauen Zielregion am Kopf fixiert werden. Entsprechend dem Krankheitsbild werden bestimmte Informationen, die für die Tiefenhirnstimulation relevant sind, wie etwa die Wahl eines speziellen Stimulationsprogramms und/oder die genauen Koordinaten der zu behandelnden Zielregion in die Planungsstation eingegeben. Anschließend werden die Elektroden bezüglich der Zielregion registriert, d.h. die relative Lage von Zielregion einerseits und Elektroden andererseits wird ermittelt bzw. eine Korrelation zwischen Elektroden und Zielregion ermittelt. Schließlich werden je nach Therapieansatz die Anzahl der verwendeten Wechselstromsignale sowie deren Parameter wie Dauer der Stimulation, zeitliche Abfolge der Therapiephasen, Frequenzen der Signale, Amplituden, Signalformen sowie Pulsfolgen der Elektrostimulation eingegeben. Vorzugsweise führt der Controller vorher festgelegte Therapieprogramme aus, so dass an der Planungsstation lediglich das Krankheitsbild und/oder der Therapieansatz zugegeben ist.

In Abhängigkeit der eingegebenen Koordinaten der Zielregion ermittelt der Controller 9 wenigstens eine Paarung von Elektroden, deren Trajektorie durch das zu stimulierende Arial im Hirn verläuft. Wie Fig. 1 zu sehen, verläuft die Trajektorie 5 des mit dem Bezugszeichen 3c/3i angegebenen Elektrodenpaares durch das Zielareal 6. In Abhängigkeit von dem beabsichtigten Therapieansatz wird das Zielareal 6 entsprechend stimuliert. So können z.B. mehrere Paarungen von Elektroden die das Zielareal kreuzen, wie z.B. auch die Paarung 3f/3o gleichzeitig oder sequentiell bzw. nacheinander verwendet werden, und zwar jeweils mit identischen oder aber auch mit unterschiedlichen Signalformen, Amplituden und Frequenzen. Die verschiedenen Wechselstromsignale werden von den mehreren unabhängigen Funktionsgeneratoren 2a, 2b bereitgestellt.

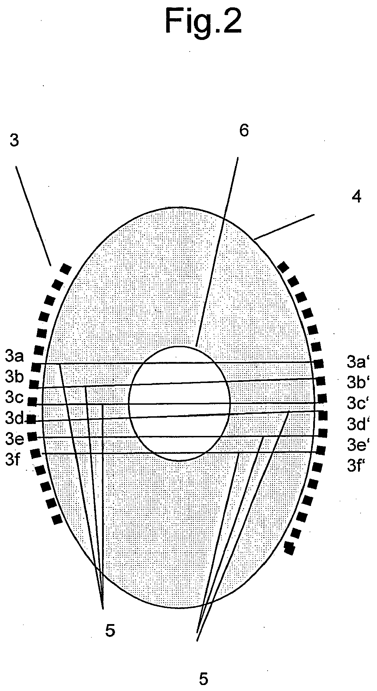

In den Figuren 2 bis 4 sind verschiedene Varianten zur kortikal lokal fokussierten Therapie jeweils in Ansichten von oben des Kopfes 4 einer zu behandelnden Person schematisch dargestellt. In Fig. 2 sind gemäß einer ersten Variante die Elektrodenpaare derart gewählt, dass die Trajektorien 5 dieser Elektrodenpaare, nämlich die Paarungen 3a/3a', 3b/3b', 3c/3c', 3d/3d', 3e/3e' & 3f/3f', das Zielareal 6 kreuzen und im Wesentlichen parallel zueinander verlaufen. Diese Variante ist besonders vorteilhaft, wenn möglichst viele, eng benachbarte Elektroden an dem Kopf 4 der zu behandelnden Person platziert sind.

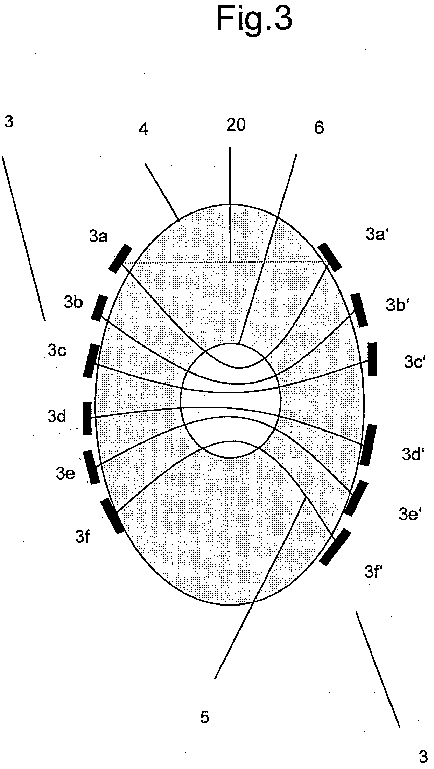

Fig. 3 zeigt eine zweite Variante, bei der die Elektroden in größeren Abständen in Umfangsrichtung um den Kopf 4 angeordnet sind. Bei dieser Variante werden diejenigen Elektroden 3a-3l als Elektrodenpaarung ausgewählt, die auf gegenüberliegenden Seiten des Kopfes 4 angeordnet sind und den geringsten Abstand zueinander aufweisen. Der zwischen den jeweiligen Elektrodenpaaren 3a/3a', 3b/3b', 3c/3c', 3d/3d', 3e/3e' & 3f/3f' fließende Strom wird in Abhängigkeit von dem elektrischen Widerstandsverhalten des Widerstands, der sich aus dem Übergangswiderstand zwischen der Elektrodenfläche und der Kopfhaut sowie den Widerstandseigenschaften des Schädels und des Hirns ergibt, so eingestellt, dass auch die Trajektorien 5 von Elektrodenpaaren, deren Abstandslinien normalerweise nicht durch das Zielareal sondern im Wesentlichen entlang einer (in Fig. 3 strichpunktiert angegebenen) direkten Abstandslinie 20 verlaufen würden, bogenförmige und insbesondere parabelförmige Verläufe annehmen, und zwar derart, dass auch die Trajektorien 5 dieser Paare das Zielareal 6 kreuzen, wie in Fig. 3 zu sehen ist.

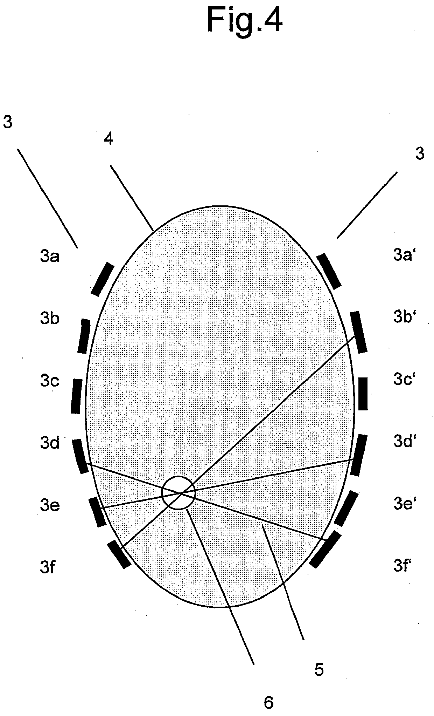

Fig. 4 zeigt eine dritte Variante, bei der genau diejenigen Paarungen von Elektroden, nämlich 3d/3f', 3e/3d' und 3f/3b', ausgewählt werden, deren Trajektorien 5 das Zielareal 6 kreuzen und deren Trajektorien 5 wiederum sich in dem Zielareal 6 kreuzen. Bei dieser Variante ist besonders vorteilhaft, dass im Zielareal eine größtmögliche Stimulation erreicht werden kann, während gleichzeitig das umliegende Gehirnareal minimal stimuliert wird. Dementsprechend wird die Energiezufuhr auf das zu behandelnde Areal fokussiert und umgebende Bereiche des Gehirns nur geringfügig beeinflusst. Darüber hinaus können bei dieser Variante trotz einer geringeren Anzahl von Elektroden sämtliche Areale im Gehirn erreicht werden.

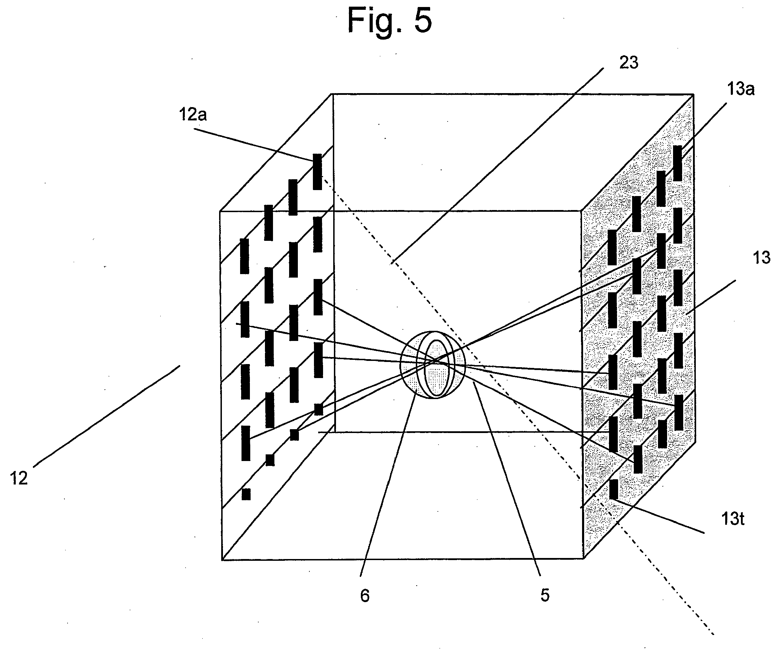

In Fig. 5 ist das Prinzip der kortikal lokal fokussierten Therapie mittels eines Elektrodenarray-Paares 12, 13 in einer dreidimensionalen Ansicht schematisch dargestellt. Zur einfacheren Darstellung sind lediglich die beiden Elektrodenarrays 12, 13, die jeweils 20 einzelne Elektroden umfassen, sowie das Zielareal 6 des Hirns gezeigt. Jedes Elektrodenarray kann aber auch 64 und vorzugsweise 128 Elektroden umfassen. Wie durch die durchgezogenen Linien angedeutet, sind gemäß diesem Beispiel genau vier Elektrodenpaarungen ausgewählt worden, deren Trajektorien 5 sowohl das Zielareal 6 als auch die jeweils anderen Trajektorien in dem Zielareal 6 kreuzen. Die Elektrodenarrays 12, 13 sind matrix- bzw. rasterartig in Zeilen und Spalten angeordnet, es können aber auch andere Anordnungen verwendet werden, die z.B. je nach dem zu behandelnden Zielareal verschieden gebildet sind und Bereiche höherer und geringerer Elektrodendichte aufweisen. Wie beispielhaft durch die strichpunktierte Linie 23 angegeben, kann die Elektrode 12a keine Paarung mit einer der Elektrode 13a-13t bilden, deren Trajektorie das Zielareal 6 kreuzt.

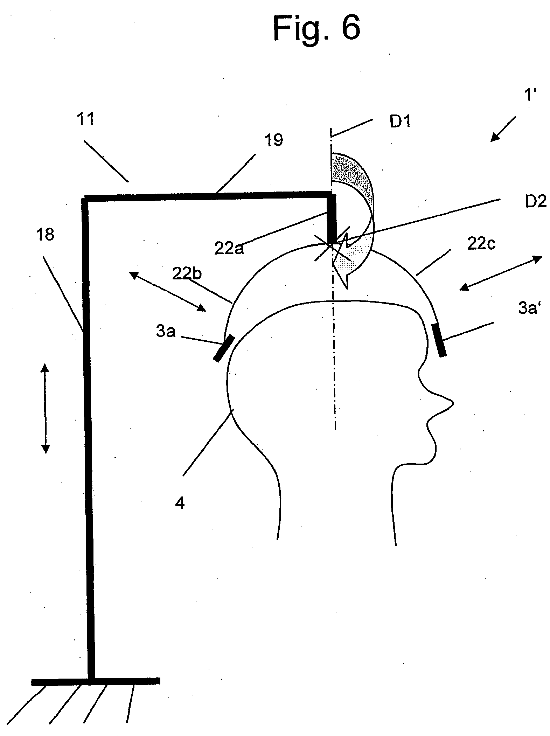

Fig. 6 zeigt eine zweite bevorzugte Ausführungsform des erfindungsgemäßen Tiefenhirnstimulators. Der Tiefenhirnstimulator 1' umfasst eine Positioniervorrichtung 11, die in Form einer navigierbaren mechatronischen Vorrichtung vorgesehen ist. An der Positioniervorrichtung 11 ist ein Elektrodenpaar 3a, 3a' angeordnet. Die Positioniervorrichtung 11 umfasst eine Halterung, die als eine senkrecht stehende und an einer feststehenden Auflage angeordnete höhenverstellbare, insbesondere teleskopierbare, Stützstange 18 besteht. Am oberen Ende der Stützstange ist ein Auslgerarm 19 befestigt, der vorzugsweise in einem im Wesentlichen rechten Winkel in horizontaler Richtung absteht. An dem distalen Ende des Auslegerarms 19 ist eine Elektrodenhalterung 22 angebracht. Die Elektrodenhalterung 22 umfasst einen Dreharm 22a, der von dem distalen Ende des Auslegerarms 19 senkrecht nach unten abragt und um eine vertikale Drehachse D1 drehbar gelagert ist. Die Elektrodenhalterung 22 umfasst weiterhin zwei Schwenkarme 22b, 22c, die sich von dem unteren Ende des Dreharms 22a radial nach außen erstrecken. An den distalen Enden der Schwenkarme 22a, 22b sind die Elektroden 3a, 3a' mit deren Kontaktflächen nach innen in Richtung des zu behandelnden Kopfes weisend befestigt. Die Schwenkarme 22b, 22c sind in Richtung nach unten bogenförmig gebildet, um die Elektroden 3a, 3a' im Bereich seitlich des Schädels platzieren zu können.

Weiterhin sind die Schwenkarme 22b, 22c jeweils um eine horizontale Drehachse D2 schwenkbar an dem unteren Ende des Dreharms 22a befestigt, so dass die Schwenkarme 22a, 22b eine Greifanordnung bilden. Durch die Greifbewegung können die Elektroden 3a, 3a' an die Außenseite des Kopfes 4 angelegt und von dieser wieder wegbewegt werden. Durch die Dreheigenschaft der Greifanordnung um eine vertikale Achse D1 kann das Elektrodenpaar 3a, 3a' in verschiedenen Drehpositionen am Kopf 4 angeordnet werden.

Im Betrieb sind die Elektroden 3a, 3a' an einen (nicht gezeigten) Funktionsgenerator anschlossen. Sowohl der Funktionsgenerator als auch die Positioniervorrichtung 11 sind an eine (nicht gezeigte) Stromapplikations-Steuerungseinrichtung angeschlossen, welche den Positioniervorgang durch die Positioniervorrichtung sowie die Stromapplikation an den Elektroden 3a, 3a' steuert Somit können nacheinander Drehpositionen angefahren werden, in denen das Elektrodenpaar 3a, 3b nach dem Heranschwenken zum Platzieren an der Kopfhaut mit einem kurzzeitigen, gepulsten Wechselstrom beaufschlagt werden kann, dessen Trajektorie das Zielareal kreuzt. Bevorzugt werden nacheinander solche Drehpositionen angefahren, in denen sich die Trajektorien der applizierten Wechselströme im Zielareal kreuzen. Vorzugsweise sind die Schwenkarme 22b, 22c teleskopierbar, so dass deren Länge je nach Lage die Zielareals 6 in dem Kopf 4 variabel einstellbar sind. Die aus Fig. 1 bekannten Komponenten wie Controller, Planungsstation & Bio-Feedback-Einrichtung sind auch auf den in Fig. 6 gezeigten Tiefenhirnstimulator übertragbar.

## Patentansprüche

1. Vorrichtung (1) zur transkraniellen Wechselstromstimulation (tASC) von tiefen Hirnarealen, aufweisend:
- wenigstens einen Signalgenerator (2a, 2b) zur Erzeugung eines elektrischen Wechselstromsignals,
- eine Elektroden-Anordnung (3), die an dem Kopf (4) einer zu behandelnden Person platzierbar und mit dem Signalgenerator (2a, 2b) elektrisch verbindbar ist, um ein Wechselstromsignal zu applizieren,
wobei
mittels der Elektroden-Anordnung (3) wenigstens zwei Wechselstromsignale applizierbar sind, deren Trajektorien (5) das zu behandelnde Hirnareal (6) kreuzen, wobei das Wechselstromsignal hochfrequent ist und eine Frequenz von zwischen 1 kHz und 50 kHz hat, **dadurch gekennzeichnet, dass**
die Elektroden-Anordnung (3) wenigstens zwei Elektrodenpaare (3a-p, 3a-p; 3a-f, 3a'-f'; 12a-t, 13a-t) mit jeweils einer Ziel- (A) und einer Referenzelektrode (K) umfasst, wobei die wenigstens zwei Elektrodenpaare (3a-p, 3a-p; 3a-f, 3a'-f'; 12a-t, 13a-t) derart räumlich positionierbar sind, dass sich die Trajektorien (5) wenigstens zweier applizierter Stromsignale in dem zu behandelnden Hirnareal (6) kreuzen und eine Stromapplikations-Steuerungseinrichtung (7, 7') zur Steuerung der an den wenigstens zwei Elektrodenpaaren (3a-p, 3a-p; 3a-f, 3a'-f'; 12a-t, 13a-t) applizierten Stromsignalen bereitgestellt ist, die derart betreibbar ist, dass die wenigstens zwei Stromsignale sequentiell, insbesondere wechselseitig, appliziert werden.

2. Vorrichtung (1) nach Anspruch 1,
**dadurch gekennzeichnet, dass**
das Wechselstromsignal ein gepulstes Wechselstromsignal ist.

3. Vorrichtung (1) nach einem der vorhergehenden Ansprüche,
**dadurch gekennzeichnet, dass**
die elektrischen Stimulationsparameter wie Frequenz, Amplitude, Kurvenform und Pulsfolge des Stromsignals veränderbar sind.

4. Vorrichtung nach einem der vorhergehenden Ansprüche,
**dadurch gekennzeichnet, dass**
die Elektroden-Anordnung (3) ein Elektrodenarray-Paar (12; 13) umfasst, wobei jedes Elektrodenarray (12; 13) eine Vielzahl von rasterartig bzw. matrixartig angeordneten Elektroden (12a-12t; 13a-13t) umfasst, wobei jeweils ein Elektrodenarray (12) als die Ziel- (A) und ein Elektrodenarray (13) als die Referenzelektrode (K) fungiert, und wobei die Anzahl der Zielelektroden der Anzahl der Referenzelektroden entspricht.

5. Vorrichtung nach Anspruch 4,
**dadurch gekennzeichnet, dass**
die Stromapplikations-Steuerungseinrichtung (7) selektiv betreibbar ist, um diejenigen Paarungen von Elektroden aus der Vielzahl von Elektroden auszuwählen, deren Stromsignal-Trajektorien sich in dem zu behandelnden Gehirnareal kreuzen.

6. Vorrichtung nach einem der vorhergehenden Ansprüche,
**dadurch gekennzeichnet, dass**
die Stromapplikations-Steuerungseinrichtung (7) einen Matrix-Schalter (8) und eine Controller-Einrichtung (9), die den Matrix-Schalter (8) ansteuert, umfasst.

7. Vorrichtung nach einem der vorhergehenden Ansprüche,
**dadurch gekennzeichnet, dass**
ein Befestigungsmittel vorgesehen ist, um das Elektrodenpaar bzw. die Elektrodenarrays (12; 13) an dem Kopf, insbesondere der Kopfhaut, der zu behandelnden Person zu fixieren.

8. Vorrichtung nach Anspruch 7,
**dadurch gekennzeichnet, dass**
es sich bei dem Befestigungsmittel um einen Gurt, eine Kopfbedeckung, oder eine Kappe handelt, und die Elektroden₋Arrays (12; 13) oder die Elektroden (3a, 3b,...; 3a', 3b',...) in dem Befestigungsmittel integriert sind.

9. Vorrichtung nach einem der vorhergehenden Ansprüche,
**dadurch gekennzeichnet, dass**
die Stromapplikations-Steuerungseinrichtung (7') eine Positioniervorrichtung (11) umfasst, an der ein Elektrodenpaar (3a, 3a') vorgesehen ist, wobei die Positioniervorrichtung (11) betreibbar ist, um das Elektrodenpaar (3a, 3a') an dem Kopf der zu behandelnden Person in einer ersten Elektrodenpaar-Stellung sowie in einer zweiten Elektrodenpaar-Stellung zu positionieren, wobei die erste und die zweite räumliche Elektrodenpaar-Stellung derart zueinander versetzt sind, dass sich die Trajektorien (5) der in der ersten und der zweiten Elektrodenpaar-Stellung applizierten Stromsignale in dem zu behandelnden Hirnareal (6) kreuzen, und die Stromapplikations-Steuerungseinrichtung (7') eine Controller-Einrichtung (9) umfasst, die den Betrieb der Positioniervorrichtung (11) sowie den Betrieb des Funktionsgenerators (2a, 2b) steuert, um die Positionierung des Elektrodenpaares (3a, 3a') und die Stromapplizierung in den verschiedenen Elektrodenpaar-Stellungen abzustimmen.

10. Vorrichtung nach einem der vorhergehenden Ansprüche,
**dadurch gekennzeichnet, dass**
weiterhin eine Planungsstation (15) zur Bestimmung von Lage und Ausmaße der zu stimulierenden Areale des Gehirns vorgesehen ist, und die Bestimmung durch volumetrische anatomische Bilddaten wie z.B. CT oder MRT erfolgt.

11. Vorrichtung nach einem der vorhergehenden Ansprüche,
**dadurch gekennzeichnet, dass**
eine Feedback-Einrichtung (16) vorgesehen ist, um Feedbackdaten betreffend den Therapieerfolg und der medizinischen Befindlichkeit des Patienten der Controller-Einrichtung (9) zur weiteren Auswertung und Optimierung der Stimulationsparameter zuzuführen.

12. Vorrichtung nach einem der vorhergehenden Ansprüche,
**dadurch gekennzeichnet, dass**
die Elektroden (3a.., 3a'..) flächig ausgebildet sind und vorzugsweise eine Fläche von wenigen mm² bis wenigen cm² aufweisen.

13. Vorrichtung nach einem der vorhergehenden Ansprüche 1 bis 12 zur Verwendung in einem Verfahren zur Behandlung von Funktionsstörungen des Gehirns.

## Claims

1. An apparatus (1) for transcranial alternating current stimulation (tACS) of deep brain areas, comprising:
- at least one signal generator (2a, 2b) for generating an electrical alternating-current signal,
- an electrode arrangement (3) which can be placed on the head (4) of a person to be treated and to which the signal generator (2a, 2b) can be connected electrically so as to apply an alternating-current signal,
wherein the electrode arrangement (3) can be used to apply at least two alternating-current signals whose trajectories (5) cross the region of the brain (6) to be treated, wherein the alternating-current signal is of high frequency and has a frequency of between 1 kHz and 50 kHz, **characterized in that** the electrode arrangement (3) comprises at least two pairs of electrodes (3a-p, 3a-p; 3a-f, 3a'-f'; 12a-t, 13a-t) with one respective target (A) and reference electrode (K), wherein the at least two pairs of electrodes (3a-p, 3a-p; 3a-f, 3a'-f'; 12a-t, 13a-t) can be positioned spatially in such a way that the trajectories (5) of at least two applied current signals cross in the brain area (6) to be treated, and a current application control device (7, 7') is provided for controlling the current signals applied to the at least two pairs of electrodes (3a-p, 3a-p; 3a-f, 3a'-f'; 12a-t, 13a-t), which can be operated in such a way that the at least two current signals are applied sequentially, especially reciprocally.

2. An apparatus (1) according to claim 1, **characterized in that** the alternating-current signal is a pulsed alternating-current signal.

3. An apparatus (1) according to one of the preceding claims, **characterized in that** the electrical stimulation parameters such as frequency, amplitude, curve shape and pulse sequence of the current signal are variable.

4. An apparatus according to one of the preceding claims, **characterized in that** the electrode arrangement (3) comprises a pair of electrode arrays (12; 13), wherein each electrode array (12; 13) comprises a plurality of electrodes (12a-12t; 13a-13t) arranged in a grid-like or matrix-like manner, wherein one respective electrode array (12) acts as the target electrode (A) and one electrode array (13) as the reference electrode (K), and wherein the number of the target electrodes corresponds to the number of the reference electrodes.

5. An apparatus according to claim 4, **characterized in that** the current application control device (7) can be operated selectively in order to select such pairs of electrodes from the plurality of electrodes whose current signal trajectories cross in the brain area be treated.

6. An apparatus according to one of the preceding claims, **characterized in that** the current application control device (7) comprises a matrix switch (8) and a controller device (9) which triggers the matrix switch (8).

7. An apparatus according to one of the preceding claims, **characterized in that** a fastening means is provided in order to fix the pair of electrodes or the electrode arrays (12; 13) to the head, especially the scalp, of the person to be treated.

8. An apparatus according to claim 7, **characterized in that** the fastening means concerns a strap, a head cover or a cap, and the electrode arrays (12; 13) or electrodes (3a, 3b, ....; 3a', 3b', ...) are integrated in the fastening means.

9. An apparatus according to one of the preceding claims, **characterized in that** the current application control device (7') comprises a positioning apparatus (11) on which a pair of electrodes (3a, 3a') are provided, wherein the positioning device (11) can be operated to position the pair of electrodes (3a, 3a') on the head of the person to be treated in a first position of the pair of electrodes and in a second position of the pair of electrodes, wherein the first and the second position of the pairs of electrodes are offset with respect to each other in such a way that the trajectories (5) of the current signals applied in the first and the second position of the pairs of electrodes cross in the brain area (6) to be treated, and the current application control device (7') comprises a control device (9) which controls the operation of the positioning device (11) and the operation of the functional generator (2a, 2b) in order to adjust the positioning of the pair of electrodes (3a, 3a') and the current application in the different positions of the pairs of electrodes.

10. An apparatus according to one of the preceding claims, **characterized in that** furthermore a planning station (50) is provided for determining the position and the dimensions of the areas of the brain to be stimulated, and the determination occurs by volumetric anatomic image data such as CT or MRT.

11. An apparatus according to one of the preceding claims, **characterized in that** a feedback device (16) is provided in order to supply feedback data concerning the success of therapy and the medical condition of the patient to the control device (9) for further evaluation and optimisation of the stimulation parameters.

12. An apparatus according to one of the preceding claims, **characterized in that** the electrodes (3a, .., 3a' ..) are formed in a flat manner and preferably have an area of a few mm² to a few cm².

13. An apparatus according to one of the preceding claims 1 to 12 for use in a method for treating functional disorders of the brain.

## Revendications

1. Appareil (1) pour la stimulation transcrânienne de zones cérébrales profondes au moyen de courants alternatifs (tASC), comprenant :
- au moins un générateur de signaux (2a, 2b) pour engendrer un signal électrique à courant alternatif,
- un agencement d'électrodes (3) qui peuvent être placées sur la tête (4) d'une personne à traiter et qui peuvent être connectées électriquement avec le générateur de signaux (2a, 2b), afin d'appliquer un signal à courant alternatif,
dans lequel au moyen de l'agencement d'électrodes (3) au moins deux signaux à courant alternatif peuvent être appliqués, dont les trajectoires (5) croisent la zone cérébrale (6) à traiter, dans lequel le signal à courant alternatif est un signal à haute fréquence et a une fréquence entre 1 kHz et 50 kHz,
**caractérisé en ce que**
l'agencement d'électrodes (3) inclut au moins deux paires d'électrodes (3a-p, 3a-p ; 3a-f, 3a'-f ; 12a-t, 13a-t) avec respectivement une électrode cible (A) et une électrode de référence (K), dans lequel lesdites au moins deux paires d'électrodes (3a-p, 3a-p ; 3a-f, 3a'-f' ; 12a-t, 13a-t) sont susceptibles d'être positionnées dans l'espace de telle façon que les trajectoires (5) d'au moins deux signaux électriques appliqués se croisent dans la zone cérébrale (6) à traiter, et il est prévu un dispositif de commande d'application de courant (7, 7') pour la commande des signaux électriques appliqués auxdites au moins deux paires d'électrodes (3a-p, 3a-p ; 3a-f, 3a'-f' ; 12a-t, 13a-t), dispositif qui peut être amené à fonctionner de telle façon que lesdits au moins deux signaux électriques sont appliqués de manière séquentielle, et en particulier en alternance.

2. Appareil (1) selon la revendication 1,
**caractérisé en ce que** le signal à courant alternatif est un signal à courant alternatif pulsé.

3. Appareil (1) selon l'une des revendications précédentes,
**caractérisé en ce que** les paramètres de stimulation électrique comme la fréquence, l'amplitude, la forme de courbe, et la succession d'impulsions du signal électrique sont modifiables.

4. Appareil selon l'une des revendications précédentes,
**caractérisé en ce que** l'agencement d'électrodes (3) inclut une paire de réseaux d'électrodes (12 ; 13), dans lequel chaque réseau d'électrodes (12 ; 13) inclut une pluralité d'électrodes (12a-12t ; 13a-13t) agencées suivant une trame ou suivant une matrice, dans lequel un réseau d'électrodes (12) fait respectivement office d'électrode cible (A) et un réseau d'électrodes (13) fait office d'électrode de référence (K), et dans lequel le nombre des électrodes cible correspond au nombre des électrodes de référence.

5. Appareil selon la revendication 4,
**caractérisé en ce que** le dispositif d'application et de commande de courant (7) est susceptible de fonctionner de manière sélective, afin de sélectionner celles des paires d'électrodes, parmi la pluralité d'électrodes, dont les trajectoires du signal électrique se croisent dans la zone cérébrale à traiter.

6. Appareil selon l'une des revendications précédentes,
**caractérisé en ce que** le dispositif d'application et de commande de courant (7) inclut un commutateur matriciel (8) et un système contrôleur (9) qui pilote le commutateur matriciel (8).

7. Appareil selon l'une des revendications précédentes,
**caractérisé en ce qu'**il est prévu un moyen de fixation pour fixer la paire d'électrodes ou les réseaux d'électrodes (12 ; 13) sur la tête, en particulier sur le derme de la tête de la personne à traiter.

8. Appareil selon la revendication 7,
**caractérisé en ce que** le moyen de fixation est une ceinture, un couvrechef ou un bonnet, et les réseaux d'électrodes (12 ; 13) ou les électrodes (3a, 3b, ... ; 3a', 3b', ...) sont intégré(e)s dans le moyen de fixation.

9. Appareil selon l'une des revendications précédentes,
**caractérisé en ce que** le dispositif d'application de courant et de commande (7') inclut un dispositif de positionnement (11) sur lequel est prévue une paire d'électrodes (3a, 3a'), dans lequel le dispositif de positionnement (11) est susceptible de fonctionner afin de positionner la paire d'électrodes (3a, 3a') sur la tête de la personne à traiter dans une première position de paire d'électrodes ainsi que dans une seconde position de paire d'électrodes, dans lequel la première et la seconde position de paire d'électrodes sont décalées dans l'espace l'une par rapport à l'autre de telle façon que les trajectoires (5) des signaux électriques appliqués dans la première position et dans la seconde position de paire d'électrodes se croisent dans la zone cérébrale (6) à traiter, et le dispositif d'application de courant et de commande (7') inclut un système contrôleur (9), qui pilote le fonctionnement du dispositif de positionnement (11) ainsi que le fonctionnement du générateur fonctionnel (2a, 2b) afin d'accorder le positionnement de la paire d'électrodes (3a, 3a') et l'application du courant dans les différentes positions des paires d'électrodes.

10. Appareil selon l'une des revendications précédentes,
**caractérisé en ce qu'**il est en outre prévu un poste de planification (15) pour la détermination de la position et des dimensions des zones du cerveau à stimuler, et la détermination a lieu au moyen de données d'imagerie anatomique volumétrique, comme par exemple CT ou MRT.

11. Appareil selon l'une des revendications précédentes,
**caractérisé en ce qu'**il est prévu un dispositif de rétroaction (16) afin d'envoyer au système contrôleur (9) des données de rétroaction concernant le succès thérapeutique et la sensibilité médicale du patient, pour la poursuite de l'évaluation et l'optimisation des paramètres de stimulation.

12. Appareil selon l'une des revendications précédentes,
**caractérisé en ce que** les électrodes (3a ..., 3a' ...) sont réalisées de manière surfacique et présentent de préférence une surface de quelques millimètres carrés à quelques centimètres canés.

13. Appareil selon l'une des revendications 1 à 12, destiné à l'utilisation dans un procédé pour le traitement de perturbations fonctionnelles du cerveau.
